# EUROPEAN PATENT SPECIFICATION

(11) **EP 2 827 848 B1**
(45) Date of publication and mention of the grant of the patent: **27.04.2016**
(21) Application number: 12712618.3
(22) Date of filing: 21.03.2012
(51) Int. Cl.: A61K 31/135, A61P 25/28, A61P 25/00, A61P 25/16, A61K 9/20, A61K 31/194

(54) **STABILIZED PHARMACEUTICAL COMPOSITIONS COMPRISING RASAGILINE SALTS**
STABILIZIERTE PHARMAZEUTISCHE ZUBEREITUNGEN ENTHALTEND SALZE VON RASIGILINE
COMPOSITIONS PHARMACEUTIQUES STABLES COMPRENANT DES SELS DE LA RASILIGINE

(43) Date of publication of application: 28.01.2015
(73) Proprietor: Synthon BV, 6545 CM Nijmegen (NL)
(72) Inventor: ÁLVAREZ FERNÁNDEZ, Lisardo, E-08830 Sant Boi de Llobregat (ES); ELFFRINK, Walter, Wilhelmus, Johannes, NL-6545 CM Nijmegen (NL)
(74) Representative: Mendivil Gil, Maria Dolores
(86) International application number: PCT/EP2012/054973
(87) International publication number: WO 2013/139387

(56) References cited:
- WO-A1-02/068376
- US-A1- 2010 234 636
- US-A1- 2011 152 381
- US-A1- 2011 313 050

## Description

### BACKGROUND OF THE INVENTION

Rasagiline, R-(+)-N-2-Propynyl-1-indanamine of the formula (1) 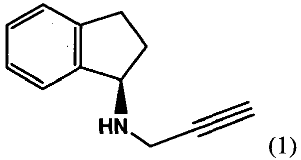 is a potent, selective, irreversible monoamine oxidase-type B (MAO-B) inhibitor for the treatment of Parkinson's disease, Alzheimer disease and various types of dementia. The compound was disclosed in EP 436492. Solid, particularly crystalline state rasagiline was disclosed in WO 2008/076348, WO 2009/154777.

Rasagiline is the R-enantiomer of known N-2-Propynyl-1-indanamine. The R-orientation of the propargylamino- moiety is responsible for the desired pharmaceutical effect as the R-enantiomer is pharmaceutically far more potent in the inhibition of MAO-B than the S-enantiomer (see, e.g., WO 95/11016 for further discussion of this topic).

Rasagiline contains one basic nitrogen and accordingly it may form acid addition salts. At present, the acid addition salts are preferred for pharmaceutical applications.

In the marketed pharmaceutical formulations, the compound (1) is present as the methane sulfonate salt (i.e., rasagiline mesylate). The marketed tablets, sold under the brand name AZILECT® (Teva Pharmaceutical Industries), comprise 0.5 or 1 mg of the rasagiline mesylate and excipients of mannitol, starch, pregelatinized starch, colloidal silica, stearic acid and talc. Rasagiline mesylate was originally disclosed in WO 95/11016 and WO 96/37199.

Rasagiline mesylate, as well as compositions comprising it, of various states and degrees of purity were furthermore disclosed in US 7572834, WO 2009/118657, WO 2009/122301, WO 2009/141737, WO 2010/013048.

The original salt form of rasagiline, which was studied in pharmaceutical and toxicological tests, was rasagiline hydrochloride. Rasagiline hydrochloride was also disclosed in EP 436492. Subsequently some stability problems were reported for rasagiline hydrochloride (see, e.g., discussion in WO 2006/057912 and particularly Example 32 of EP 812190) and a switch was made to the mesylate salt.

Crystalline polymorphs of rasagiline hydrochloride were disclosed in US 2010/010098 and in WO 2010/007181.

A tannate salt of rasagiline has been disclosed in WO 2008/076315. The rasagiline tannate compositions are currently in preclinical evaluation.

Acid addition salts of rasagiline with organic diacids, specifically rasagiline edisylate and rasagiline oxalate have been disclosed in WO 2008/019871. Also these two salts are in preclinical evaluations.

Several other acid addition salts of rasagiline were disclosed in documents of the prior art. Thus, rasagiline (hemi)-L-tartrate was disclosed in WO 95/11016, US appl. 2003/0065038, WO 2007/061717, US Appl. 2010/0029987 or WO 2010/007181.

The WO 2010/007181 furthermore disclosed many other crystalline rasagiline salts, i.e. a fumarate salt, succinate salt, besylate salt etc.

WO 2010/085354 discloses a citrate salt, especially in an amorphous form, but relates to a delayed release rasagiline formulation.

An important aspect in practical application of rasagiline and/or its salts is a problem of obtaining a sufficiently stable pharmaceutical composition. So far, it appears that the most stable compositions are compositions comprising a large amount of mannitol as the stabilizing excipient, as shown in EP0858328 or US 6126968. Also the marketed AZILECT product comprises mannitol in the composition.

In addition, WO 2006/091657 reports problems resulting from the fact that the homogeneous distribution of rasagiline in a pharmaceutical product cannot be fully guaranteed due to relatively low loading of the active. In order to solve the problem, WO 657 proposes to use active ingredient particles of a certain size.

While pharmaceutical compositions comprising rasagiline are known in the art, an improvement in the matter is still desirable. In particular, it would be beneficial to find ways to prepare stabilized pharmaceutical composition with other rasagiline salts than mesylate, which would extend the spectrum of use of rasagiline-type compounds.

### BRIEF DESCRIPTION OF THE PRESENT INVENTION

The present invention deals with processes of making stable and content uniform solid pharmaceutical compositions comprising a salt of rasagiline with L-tartaric acid as the active ingredient. In particular, the invention provides a process of stabilization of pharmaceutical compositions comprising rasagiline, said process comprising combining 1 weight part of rasagiline L-tartrate with 1-5 weight parts of L-tartaric acid. Such stabilized combination may then be used in making pharmaceutical compositions comprising rasagiline L-tartrate.

In a first aspect, the invention provides a pharmaceutical composition comprising 1 weight part of rasagiline L-tartrate, 1-5 weight parts of L-tartaric acid, and at least one inert pharmaceutically acceptable excipient. The composition is preferably designed for oral administration.

In a second aspect, the invention provides a process for making pharmaceutical compositions comprising rasagiline, said process comprising combining 1 weight part of rasagiline L-tartrate with 1-5 weight parts of L-tartaric acid.L-tartrate. In an advantageous embodiment, said process comprises the steps of
a) dissolving 1 weight part of rasagiline L-tartrate and 1-5 weight parts of L-tartaric acid in water; and
b) combining the obtained solution with at least one inert solid carrier.

In particular, the above process may be used in making a pharmaceutical granulate, preferably wherein the solution obtained in step a) serves as a granulation liquid during step b).

Accordingly, the invention also provides a granulate comprising 1 weight part of rasagiline L-tartrate, 1-5 weight parts of L-tartaric acid, and at least one inert solid carrier. In a particular aspect, the invention provides a granulate obtainable by combining an aqueous solution of 1 weight part of rasagiline L-tartrate with 1-5 weight parts of L-tartaric acid, with at least one inert solid carrier, and granulating said mixture.

In a third aspect, the invention provides a compressed tablet dosage form for oral administration comprising a unit dose amount of a composition comprising 1 weight part of rasagiline L-tartrate, 1-5 weight parts of L-tartaric acid, and at least one inert pharmaceutically acceptable excipient.

### DETAILED DESCRIPTION OF THE INVENTION

The present invention relates to stabilized pharmaceutical compositions comprising rasagiline L-tartrate as the active ingredient, and L-tartaric acid as a stabilizer.

Within the invention, the name "rasagiline" is used as a generic name for (R)-N-2-Propynyl-1-indanamine ( (R)-N-propargyl-1-aminoindan) of the formula (1)

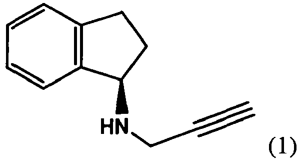

The "rasagiline L-tartrate", as used in the present invention, is a salt of rasagiline and L-(+)-tartaric acid. As L-tartaric acid is a bivalent acid, rasagiline might stoichiometrically form both 1:1 and 2:1 tartrate salts. In the prior art documents describing tartrate salts, defined compounds with 1:1 molar ratio (the alternative nomenclature is then rasagiline hydrogentartrate) or 2:1 molar ratio (the alternative nomenclature is then rasagiline hemitartrate) have been described. However the "rasagiline L-tartrate", as used in the present invention may comprise any, even a not stoichiometric, combination of rasagiline and L-tartaric acid.

The "rasagiline fumarate", in the present disclosure, is a salt of rasagiline and fumaric acid. As fumaric acid is a bivalent acid, rasagiline might stoichiometrically form both 1:1 and 2:1 fumarate salts. In the prior art documents, only a compound with 1:1 molar ratio of both components (the alternative nomenclature is then rasagiline hydrogenfumarate) has been disclosed as a defined product. However the "rasagiline fumarate", comprises any, even a not stoichiometric, combination of rasagiline and fumaric acid.

Rasagiline hemi-L-tartrate has been disclosed in WO 95/11016. The product had 2:1 molar ratio of the base and acid moieties and was reported as being anhydrous (loss on drying less than 0.1 per cent), soluble in water (33 mg/ml, almost twenty times less than the corresponding mesylate), having a melting range of about 176 °C and exhibiting pH of 5.5. Rasagiline L-tartrate was also disclosed in later documents cited above. The WO 2010/007181 also disclosed rasagiline-L-hydrogentartrate (i.e. a product of 1: 1 molar ratio of the base and acid moieties) as a crystalline compound; however no physical properties were reported.

Rasagiline fumarate has been disclosed in WO 95/11016. The product had 1:1 molar ratio of the base and acid moieties and was reported as being anhydrous (loss on drying 0.2 per cent), soluble in water (95 mg/ml, almost seven times less than the corresponding mesylate), having a melting range of about 126 °C and exhibiting pH of 3.5. A crystalline Form I rasagiline fumarate with 1:1 molar ratio of the salt and base was disclosed in WO 2010/007181.

In comparative stability studies performed in WO 95/11016, various salts of N-2-propynyl-1-indanamine were tested and the mesylate salt was proven as being the most stable. The stability data of the corresponding fumarate and L-tartrate salts, which were also prepared in that document for said purpose, have however not been published therein. Logically, it may be expected that the authors of WO'116 considered neither the fumarate nor the L-tartrate as a proper candidate for pharmaceutical applications.

Accordingly, there exist a clear prejudice that neither rasagiline fumarate nor rasagiline L-tartrate, which otherwise could be expected being quite useful in pharmaceutical applications because of being solid state, nonhygroscopic and sufficiently soluble compounds, have been regarded in the art as suitable candidates in pharmaceutical applications. Most probably some undisclosed stability problems played a role.

With a surprise, it was discovered within the present invention that it is possible to prepare stable and content uniform pharmaceutical compositions comprising rasagiline L-tartrate. This raises the rasagiline L-tartrate as becoming a good equivalent to the marketed mesylate.

Furthermore, it is well known in the art that sufficiently stable pharmaceutical compositions comprising rasagiline should comprise at least one alcohol selected from group of pentahydric or hexahydric alcohols like mannitol, xylitol and sorbitol. Such alcohol is preferably present in amounts of at least 60% of the total mass of the composition. See, e.g. EP0858328 or US 6126968 for such teaching. Also the marketed AZILECT product comprises mannitol in the composition. Within the present invention, it was surprisingly found out that a stable oral pharmaceutical composition of rasagiline, which may be, in certain embodiments, bioequivalent with AZILECT, may be prepared without a need of huge amounts of mannitol or a similar sugar-like stabilizer.

The compositions of the present invention furthermore do not require any specific particle size distribution of the starting rasagiline to assure content uniformity of the composition contrary to teaching in WO 2006/091657. Actually, the particle size of starting rasagiline compound needs not to be controlled or selected in compositions of the present invention.

The the rasagiline L-tartrate for purpose of the invention may be prepared, e.g., by contacting rasagiline base and L-tartaric acid in a suitable solvent or a mixture of solvents, precipitating the salt as a solid material in the mixture and separating the solid phase from the liquid phase. The starting rasagiline base is a known compound and may be used in an isolated form or it may be present within a reaction mixture as a product of a chemical conversion.

At least two crystalline polymorphs of rasagiline fumarate may be obtained by variation of crystalline conditions.

Rasagiline fumarate Form I is a known polymorph (WO 2010/007181), which is obtainable, e.g., by dissolving molar equivalents of rasagiline and fumaric acid in hot 2-propanol and cooling the obtained solution, advantageously in a presence of seed crystals of the Form I, under stirring to room temperature. The Form I is characterized by a XRPD pattern comprising the characteristic peaks at angles 13.44, 16.92, 20.30, 21.14, 23.58 and 28.64 +/ 0.2 degrees two theta.

Rasagiline fumarate Form II is a new form and is obtainable, e.g., by dissolving molar equivalents of rasagiline and fumaric acid in hot 2-propanol, followed by slow spontaneous cooling the hot solution. The so obtained supersaturated solution is then forced to crystallize fast by scratching the surface of the flask, e.g. by a pipette. The formed solid may be isolated from the mixture by conventional ways, e.g. by filtration or by centrifugation, washed with a suitable liquid, e.g. by 2-propanol and/or diethyl ether and dried at room temperature to a constant weight. Rasagiline fumarate Form II is also obtainable by a seeding crystallization, e.g., by dissolving molar equivalents of rasagiline and fumaric acid in hot 2-propanol, followed by cooling and addition of seeds of Rasagiline fumarate Form II. The formed solid may be isolated from the mixture by conventional ways, e.g. by filtration or by centrifugation, washed with a suitable liquid, e.g. by 2-propanol and/or diethyl ether, and dried at room temperature to a constant weight.

The Form II is characterized by a XRPD pattern comprising the characteristic peaks at angles of 9.62, 12.70, 13.76, 15.61, 16.60, 19.89, 24.09 +/ 0.2 degrees two theta.

Rasagiline fumarate may also be prepared in an amorphous state. For instance, amorphous rasagiline fumarate may be prepared by freeze drying of a solution of rasagiline fumarate in water.

Rasagiline L-tartrate may be preferably a solid crystalline hemitartrate, i.e. a salt with molar ratio between rasagiline and tartaric acid being 2:1. Such product may be prepared, e.g., by processes disclosed in WO 95/11016, US appl. 2003/0065038, WO 2007/061717, US Appl. 2010/0029987 or WO 2010/007181. In general, Rasagiline hemi-L-tartrate is obtainable by contacting rasagiline base and equivalent of tartaric acid in a suitable solvent, from which the salt precipitates after cooling, addition of seed crystals, addition of an antisolvent or by a combination of these techniques. Suitable solvents are, e.g., C1-C4 aliphatic alcohols, e.g. methanol, ethanol or isopropanol, and mixtures of the same or with other solvents. XRPD pattern of crystalline hemitartrate has been reported in the prior art (WO 2010/007181).

In addition, rasagiline L-tartrate may also be prepared in an amorphous state. For instance, amorphous rasagiline L-tartrate may be prepared by freeze drying of a solution of rasagiline L-tartrate in water.

The rasagiline fumarate is quite stable in the solid state, particularly when present in the Form I. For instance, no decomposition or changes in appearance were observed at prolonged standing of the both Forms I and II at 40°C / 75% RH for six months. Also rasagiline L-hemitartrate is similarly stable under the above conditions.

In conclusion, the crystalline rasagiline L-hemitartrate is suitable as starting materials for the processes and compositions of the present invention.

The present invention relates to the use of L-tartaric acid for stabilization of rasagiline L-tartrate in a pharmaceutical composition comprising it. Under the name "L-tartaric acid" within the invention also corresponding pharmaceutically acceptable salts of such acid are contemplated, alone or in the mixture with the free acid. Examples of pharmaceutically acceptable salts are, without limitation, sodium, potassium, calcium, magnesium, ammonium or various amine salts.

Thus, in one embodiment, the present invention relates to a process for stabilization of pharmaceutical compositions comprising rasagiline. In particular, the invention provides a process of making of pharmaceutical compositions comprising rasagiline, said process comprising combining 1 weight part of rasagiline L-tartrate with 1-5 weight parts of L- tartaric acid.

In an advantageous embodiment, this "combining" represents dissolution of both rasagiline L-tartrate and L-tartaric acid in water, advantageously at ambient temperature. Thus, the preferred process is characterized by a step of dissolving 1 weight part of rasagiline L-tartrate or fumarate with 1-5 weight parts of L-tartaric acid, in water.

The stabilized combination formed this way and, advantageously, the stabilized solution formed this way, is then used in making pharmaceutical compositions incl. solid pharmaceutical compositions. Advantageously, such solid pharmaceutical composition may be made by a wet granulation, wherein the stabilized solution disclosed above serves as a granulation liquid. Such process will be discussed in more detail below.

The use of the rasagiline salt in a solution has an advantage in assuring proper content uniformity of the pharmaceutical composition, i.e. a homogeneous distribution of (relatively low) load of rasagiline within the composition, whereby it is not necessary to select the rasagiline starting material of a proper particle size.

The present invention provides a stable solid pharmaceutical composition comprising 1 weight part of rasagiline L-tartrate, 1-5 weight parts of L-tartaric acid, and at least one inert pharmaceutically acceptable excipient. Preferably, the "inert pharmaceutically acceptable excipient" is not a pentahydric or hexahydric alcohol, a polyhydric alcohol such as maltitol or xylitol or a disaccharide such as sucrose or maltose. The composition is preferably designed for oral administration.

Advantageously, the relative weight amount of rasagiline L-tartrate in the pharmaceutical composition is 0.2 - 10 weight per cent, preferably 0.5 - 5 weight per cent, based on the total mass of the composition.

The "inert pharmaceutically acceptable excipient" may be selected from the following excipients:
- a filler and/or a binder. The examples are, without limitation, microcrystalline cellulose, starch, pregelatinized starch, lactose, polyvinylpyrrolidone, hydroxypropylmethyl cellulose, hydroxypropyl cellulose and the like; and/or
- a disintegrant. Suitable disintegrant is, without limitation, a pregelatinized starch, sodium starch glycollate, croscarmellose sodium,crospovidone and the like; and/or
- a lubricant/glidant. Suitable lubricant/glidant is, without limitation, talc, stearic acid, and the like. Preferably, the lubricant/glidant is not silicon dioxide.

The stable solid pharmaceutical composition of the invention may be prepared by various ways.

In a first alternative, the pharmaceutical composition is prepared by a wet granulation. In an advantageous embodiment, the process of making stable solid pharmaceutical composition includes the process of stabilization outlined above; in particular, it includes the process of making stabilized solution outlined above. In particular, the above process may be used in making a pharmaceutical granulate, wherein the stabilized solution serves as a granulation liquid. Thus, the invention provides a process for making pharmaceutical composition comprising rasagiline L-tartrate, said process comprising the steps of
a) dissolving 1 weight part of rasagiline L-tartrate and 1-5 weight parts of L-tartaric acid in water; optionally, suitable auxilliary component(s) may be added thereto; and
b) combining the obtained solution obtained solution with at least one solid carrier.

Typically, the solution is combined with at least one filler and/or binder and, optionally, with a disintegrant and/or an auxilliary excipient. Examples of suitable fillers and/or binders, disintegrants and auxilliary excipients may be found above.

Typically, the solution is combined with the carrier composition by means of granulation.

Thus, at least one inert solid carrier excipient, which typically is at least one solid filler and/or binder, optionally admixed with disintegrant(s) and/or an auxiliary excipient(s), is granulated, e.g., in a fluid bed granulator or a mixer/granulator, using the above solution as the granulation liquid. The granulate is then dried and screened, mixed with remaining extragranular excipients and with a lubricant and formulated into pharmaceutical dosage form. Preferably, the composition is designed to be formulated into a tablet form.

The granulate comprising rasagiline L-tartrate and L-tartaric acid may be also prepared by solid mixing of 1 weight part of rasagiline L-tartrate, 1-5 weight parts of L-tartaric acid, at least one suitable carrier, which typically is an inert filler/binder and, optionally, at least one other auxiliary excipient, and wet granulating of the mixture with the aid of a suitable granulation liquid, e.g. in a fluid bed granulator or a mixer/granulator. The suitable granulation liquid may be water, a C1-C4 aliphatic alcohol or a mixture of both.

In a second alternative, 1 weight part of rasagiline L-tartrate, 1-5 weight parts of L-tartaric acid and at least one inert solid excipient are mixed in a solid state, thoroughly blended together (which blending may also include a suitable variant of a dry granulation, for instance slugging or roller compaction), screened and optionally mixed with other excipients.

In another alternative, an excipient forming an inert core may be layered by a layer comprising the mixture of 1 weight part of rasagiline L-tartrate, and 1-5 weight parts of L-tartaric acid.

The wet granulation process outlined above, particularly the process using the stabilized solution comprising rasagiline L-tartrate and L-tartaric acid, is preferred.

Accordingly, the invention also provides a granulate comprising 1 weight part of rasagiline L-tartrate, 1-5 weight parts of L-tartaric acid, and at least one inert solid carrier. In a particular aspect, the invention provides a granulate obtainable by combining a solution of 1 weight part of rasagiline L-tartrate and 1-5 weight parts of L-tartaric acid in water, with at least one inert carrier and granulating said mixture. In such a specific granulate, an amorphous rasagiline-L-tartrate and L-tartrate is present due to the specificity of the process, whereby such amorphous nature of the both salts is advantageous because of excellent content uniformity of rasagiline within the granulate.

As a result of the stabilization by L-tartaric acid, a solid composition of rasagiline L-tartrate may be prepared (by proper combination of conditions and components), which is stable for pharmaceutical applications. In an advantageous embodiment, the amount of the usually most pronounced decomposition product 1-aminoindane is less than 0.5 per cent when such stabilized composition (and/or a dosage form comprising it) is stored at 40C/75% RH for 6 months.

Selection of excipients may affect the overall dissolution rate of rasagiline from the composition or dosage form. Advantageously, the pharmaceutical composition of the present invention exhibits dissolution of more than 90 per cent of rasagiline in 15 minutes, when tested by Ph.Eur. dissolution method using a paddle equipment at 50 rpm in 500 ml of 0.1 N HCl at 37°C. In some embodiments, the composition is bioequivalent to the marketed AZILECT tablets. A stabilized composition comprising rasagiline L-tartrate and L-tartaric acid may be also formulated as a slow-release, controlled-release or a delayed-release composition by employing proper functional excipients.

The stabilized composition of the present invention may be formulated into suitable dosage forms, preferably for oral administration, wherein the dosage form comprises a unit dose of rasagiline. The pharmaceutical dosage forms may include, but are not limited to, powders, granules, pellets, tablets and capsules. Preferred dosage form is a compressed tablet for oral administration. Thus, in a specific embodiment, the invention also provides a compressed tablet dosage form for oral administration comprising an unit dose amount of a composition comprising 1 weight part of rasagiline L-tartrate, 1-5 weight parts of L-tartaric acid, and at least one inert pharmaceutically acceptable excipient. In more particular, the compressed tablet form comprises an unit dose amount of a granulate comprising 1 weight part of rasagiline L-tartrate, 1-5 weight parts of L-tartaric acid, and at least one inert solid carrier

The "unit dose amount" in the tablet is typically an amount comprising rasagiline L-tartrate equivalent to 0.1 to 5 mg of rasagiline base. In an advantageous embodiment, the unit dose amount of rasagiline fumarate or L-tartrate in the tablet is equivalent to 0.5 or 1 mg of rasagiline base.

The compressed tablet may be prepared from the stabilized composition or granulate of rasagiline L-tartrate disclosed above by any conventional tabletting method. Optionally, the compressed tablet may be coated by a film coat.

The stabilized compositions and/or dosage forms comprising rasagiline fumarate or L-tartrate and L- tartaric acid of the present invention may be used in medicine. In particular, they may be used in such treatment, amelioration or prophylaxis of diseases, which is beneficial due to selective and irreversible inhibition of monoamine oxidase-type B. Such diseases comprise, without limitation, Parkinson's disease, Alzheimer disease and various types of dementia.

The following examples are intended to illustrate the scope of the present invention but not to limit it thereto.

### Examples

### Example 1 - Crystalline rasagiline L-hemitartrate

A 5 liter 5-necked flask was charged with rasagiline free base (341.3 g, 1.99 mol) and ethanol (1.6 liter). The solution was stirred by a mechanical stirrer and the reaction was performed under a blanket of nitrogen. Seeds were added to the mixture and a solution of L-tartaric acid (149.6 g, 0.997 mol: dissolved in about 800 ml ethanol at 55°C) was added slowly during about 3 hours. The suspension was cooled to ambient temperature, filtered, washed with ethanol, and washed with diethyl ether. The material was dried overnight at 40°C under reduced pressure. A white solid (457.6 g, 1.86 mol; 93.5 % yield) was obtained.

NMR analysis revealed that the proposed compound was obtained. XRPD analysis shown that the crystalline form is identical with the known Form I (WO 2010/007181)

### Example 2 - Crystalline rasagiline hydrogenfumarate Form I (Reference example)

Process:
a) Rasagiline free base (82 mg, 0.48 mmol), fumaric acid (56 mg, 0.48 mmol) and isopropyl alcohol (0.1 ml) were mixed. The mixture was heated to reflux and after 0.5 hours at room temperature diethyl ether (1 ml) was added to the solution. Solid material was isolated by filtration. XRPD analysis revealed that rasagiline hydrogenfumarate Form I was obtained.
b) A 100 ml flask was charged with rasagiline free base (0.528 g, 3.08 mmol), fumaric acid (0.36 g, 3.10 mmol) and isopropyl alcohol (5 ml). The mixture was heated by heatgun to reflux. The mixture was stirred at room temperature and while cooling down seeds of rasagiline hydrogenfumarate Form I were added. Then the flask was placed in a waterbath (room temperature) for 3 minutes. Solid material was isolated by filtration. The material was dried overnight at 40°C under reduced pressure. XRPD analysis revealed that rasagiline hydrogenfumarate Form I was obtained. XRPD: peaks at 9.95, 10.30, 11.36, 13.44, 14.54, 16.92, 17.49, 18.13, 18.80, 20.30, 20.72, 21.14, 21.45, 21.96, 22.47, 23.08, 23.58, 24.63, 25.76, 26.69, 27.10, 27.53, 27.98, 28.64, 29.05, 29.34, 29.70, 31.10, 31.47, 31.94, 32.64, 33.02, 34.08 +/0.2 degrees two theta
   Measurement conditions: Bruker D8 Advance with a Cu source (0,1540 nm, Kα1) and a Vantec detector. The powder pattern was recorded at 2-35 ° 2 θ.

### Example 3-Crystalline rasagiline hydrogenfumarate Form II (Reference example)

Process:
a) A 250 ml flask was charged with rasagiline free base (8.00 g, 46.4 mmol), fumaric acid (5.40 g, 46.5 mmol) and isopropyl alcohol (75 ml). The solution was stirred at room temperature for 15 minutes. Then the solution was stirred for another 4 hours at 4°C. To induce crystallization the inside of the flask was scratched with a broken Pasteurs pipette. Immediately solid material was formed. The suspension was stirred overnight. Solid material was isolated by filtration. The material was dried at 40°C under reduced pressure. Yield: 12.46 g (43.3 mmol, 93.3%). XRPD analysis revealed that rasagiline hydrogenfumarate Form II was obtained.
b) A 100 ml flask was charged with rasagiline free base (0.528 g, 3.08 mmol), fumaric acid (0.36 g, 3.10 mmol) and hot isopropyl alcohol (5 ml). The mixture was heated to reflux. The mixture was stirred at room temperature and while cooling down seeds of rasagiline hydrogenfumarate Form II were added. Then the flask was placed in a water bath (room temperature) for 3 minutes. Solid material was isolated by filtration. The material was dried overnight at 40°C under reduced pressure. XRPD: peaks at 9.62, 10.02, 10.64, 11.29, 12.70, 13.20, 13.76, 14.60, 15.61, 16.39, 16.60, 18.15, 18.62, 18.94, 19.89, 20.90, 21.62, 21.89, 22.67, 23.18, 24.09, 24.58, 25.46, 25.91, 26.18, 26.63, 26.99, 27.70, 28.15, 28.88, 29.18, 29.63, 29.93, 31.55, 31.92, 32.92, 33.62, 34.00, 34.44 +/0.2 degrees two theta
   Measurement conditions: Bruker D8 Advance with a Cu source (0,1540 nm, Kα1) and a Vantec detector. The powder pattern was recorded at 2-35 ° 2 θ.

### Example 4- Rasagiline L-hemitartrate tablets

### Composition

| **Ingredients** | **Formulation** | |
|---|---|---|
| | **(%)** | **(mg)** |
| Rasagiline L-hemitartrate | 0.685 | 1.438 |
| Microcrystalline cellulose Vivapur101 | 48.674 | 102.215 |
| Microcrystalline cellulose Vivapur102 | 25.877 | 54.342 |
| L-Tartaric acid | 1.905 | 4.000 |
| Maize starch | 9.524 | 20.000 |
| Pregelatinized starch | 9.524 | 20.00 |
| Talc | 1.905 | 4.000 |
| Stearic acid | 1.905 | 4.000 |
| **Total tablet mass** | **100.000** | **210.000** |

### Process:

Rasagiline L-hemitartatre and L-tartaric acid are dissolved in water.

Microcrystalline cellulose (Vivapur 101) and native starch are blended in a high shear mixer granulator and subsequently granulated with the rasagiline water solution. Granules are then dried in the fluid bed and milled through a 1 mm sieve.

The obtained granules are blended with microcrystalline cellulose (Vivapur102), pregelatinized starch and talc in a Bohle blender. The stearic acid is then added and blended.

The composition is compressed into tablets comprising 1.438 mg of rasagiline L-hemitartrate (equivalent to 1 mg of rasagiline base) in a rotary press machine with 11.5 x 6 mm oblong punches.

**Dissolution results in 0.1 N HCl**

| | **Average rasagiline dissolved (%)** | | | | | |
|---|---|---|---|---|---|---|
| Sample /Sampling time (min) | 0 | 5 | 10 | 15 | 30 | 45 |
| compression force 4kN | 0 | 95 | 100 | 101 | 101 | 101 |
| compression force 10kN | 0 | 90 | 100 | 102 | 102 | 102 |
| compression force 14kN | 0 | 88 | 100 | 102 | 103 | 103 |

**Content uniformity results:**

| **Content Uniformity** | | | | |
|---|---|---|---|---|
| n=15 (5 points in triplicate) | Sample Weight (mg) | Conc. (Not weight corrected) mg product/unit | C.U. (Weight corrected) (%) | C.U. (Not weight corrected) (%) |
| Mean (%) | 212.07 | 1.0515 | 104.1 | 105.1 |
| RSD (%) | 0.63 | 0.9971 | 0.8 | 1.0 |

### Stability testing:

The tablets were stored in Alu/Alu blister and in a Duplex blister as well at 40°C/75%RH for 6 months. The known impurity A (1-aminoindane), unidentified impurities and the sum of impurities were monitored by HPLC.

The results are as follows:

| | **Alu/Alu blister** | **Duplex blister** |
|---|---|---|
| Impurity A | 0.41 % | 0.48% |
| Single unidentified | 0.20 % | 0.27% |
| Total unidentified | 0.50% | 0.49% |
| Total impurities | 0.91% | 0.97% |

### Example 5 Rasagiline L-hemitartrate tablets

### Composition:

| **Ingredients** | **Formulation** | |
|---|---|---|
| | **(%)** | **(mg)** |
| Rasagiline L-hemitartrate | 0.685 | 1.438 |
| Microcrystalline cellulose Vivapur 101 | 55.817 | 117.215 |
| Microcrystalline cellulose Vivapur 102 | 25.877 | 54.342 |
| L-Tartaric acid | 1.905 | 4.000 |
| Maize starch | 2.381 | 5.000 |
| Pregelatinized starch | 9.524 | 20.00 |
| Talc | 1.905 | 4.000 |
| Stearic acid | 1.905 | 4.000 |
| **Total tablet mass** | **100.000** | **210.000** |

### Process:

Equivalent to the Example 4, batch size 650.000 tablets

### Example 6 -Rasagiline hydrogenfumarate Form I tablets (Reference example)

### Composition

| **Ingredients** | **Formulation** | |
|---|---|---|
| | **(%)** | **(mg)** |
| Rasagiline hydrogenfumarate | 0.8 | 1.68 |
| Microcrystalline cellulose Vivapur 101 | 48.56 | 101.973 |
| Microcrystalline cellulose Vivapur 102 | 25.877 | 54.342 |
| L-Tartaric acid | 1.905 | 4.000 |
| Maize starch | 9.524 | 20.000 |
| Pregelatinized starch | 9.524 | 20.00 |
| Talc | 1.905 | 4.000 |
| Stearic acid | 1.905 | 4.000 |
| **Total tablet mass** | **100.000** | **210.000** |

### Process:

Rasagiline hydrogenfumarate and L-tartaric acid are dissolved at ambient temperature in an amount of water sufficient to dissolve both components

A first part of microcrystalline cellulose (101) and maize starch are blended in a high shear mixer granulator and subsequently granulated with the solution prepared above.

The resulting granulate is dried in the fluid bed and milled through a 1 mm sieve.

The milled granulate is blended with the remaining microcrystalline cellulose (102), pregelatinized starch and talc in a Bohle blender. Stearic acid is added and the mixture is blended again.

The composition is compressed into tablets comprising 1.68 mg rasagiline hydrogenfumarate (equivalent to 1.0 mg of rasagiline base) on a rotary tablet press.

### Example 7 -Rasagiline L-hemitartrate tablets

### Composition

| **Ingredients** | **(%)** |
|---|---|
| Rasagiline L-hemitartrate | 0.7 |
| Microcrystalline cellulose Vivapur101 | 58.2 |
| Microcrystalline cellulose Vivapur102 | 35.4 |
| L-Tartaric acid | 1.9 |
| Talc | 1.9 |
| Stearic acid | 1.9 |
| **Total tablet mass** | **100.000** |

### Process:

Rasagiline L-hemitartrate and L-tartaric acid are dissolved in water. This aqueous solution is used as granulation liquid. Vivapur 101 is granulated with the water solution in a high shear mixer and then dried in a fluid bed. After milling through a Fitz mill (1.0 mm sieve), the granules are blended with Vivapur 102 and talc (30 min) and finally with the stearic acid (5 min only). The composition is compressed into tablets comprising 1.438 mg of rasagiline hemitartrate (equivalent to 1 mg of rasagiline base) in a rotary press machine with 11.5 x 6 mm oblong punches.

**Dissolution in 0.1.N HCl:**

| | | **rasagiline dissolved (%)** | | | | |
|---|---|---|---|---|---|---|
| **Sample / Sampling time (min)** | 0 | **5** | **10** | **15** | **30** | **45** |
| **1** | 0 | 81 | 98 | 104 | 106 | 106 |
| **2** | 0 | 89 | 101 | 103 | 104 | 103 |
| **3** | 0 | 84 | 100 | 104 | 106 | 107 |
| **Average** | 0 | **85** | **100** | **103** | **105** | **105** |

The invention having been described it will be obvious that the same may be varied in many ways and all such modifications are contemplated as being within the scope of the invention as defined by the following claims.

## Claims

1. A pharmaceutical composition comprising 1 weight part of rasagiline L-tartrate, 1-5 weight parts of L-tartaric acid, and at least one inert pharmaceutically acceptable excipient.

2. The composition according to claim 1, wherein the relative weight amount of rasagiline L-tartrate in the pharmaceutical composition is 0.2 - 10 weight per cent, preferably 0.5 - 5 weight per cent, based on the total mass of the composition.

3. The composition according to claim 2 or 3, which exhibits dissolution of more than 90 per cent of rasagiline in 15 minutes, when tested by Ph.Eur. dissolution method using a paddle equipment at 50 rpm in 500 ml of 0.1 N HCl at 37 °C.

4. A process of making a pharmaceutical composition according to any one of claims 1 to 3, said process comprising combining 1 weight part of rasagiline L-tartrate with 1-5 weight parts of L-tartaric acid.

5. The process according to claim 4, comprising the steps of
a) dissolving 1 weight part of rasagiline L-tartrate and 1-5 weight parts of L-tartaric acid in water, and
b) combining the obtained solution with at least one inert solid carrier.

6. The process according to claim 4 or 5, wherein the pharmaceutical composition is a pharmaceutical granulate.

7. The process according to claim 5 or 6, wherein the solution obtained in step a) serves as a granulation liquid during step b).

8. A granulate comprising 1 weight part of rasagiline L-tartrate, 1-5 weight parts of L-tartaric acid, and at least one inert solid carrier.

9. The granulate according to claim 8 obtainable by combining an aqueous solution of 1 weight part of rasagiline L-tartrate with 1-5 weight parts of L-tartaric acid, with at least one inert solid carrier, and granulating said mixture.

10. The granulate according to claim 8 obtainable by solid mixing of 1 weight part of rasagiline L-tartrate, 1-5 weight parts of L-tartaric acid, at least one inert solid carrier, and wet granulating of the mixture with the aid of a granulation liquid.

11. A compressed tablet dosage form for oral administration comprising a unit dose amount of a composition according to any one of claims 1 to 3.

12. The compressed tablet dosage form according to claim 11 comprising the granulate according to any one of claims 8 to 10.

## Patentansprüche

1. Pharmazeutische Zusammensetzung umfassend 1 Gewichtsteil Rasagilin-L-Tartrat, 1-5 Gewichtsteile L-Weinsäure und mindestens einen inerten pharmazeutisch verträglichen Hilfsstoff.

2. Zusammensetzung nach Anspruch 1, wobei die relative Gewichtsmenge von Rasagilin-L-Tartrat in der pharmazeutischen Zusammensetzung 0,2-10 Gewichtsprozent, bevorzugt 0,5-5 Gewichtsprozent basierend auf der Gesamtmasse der Zusammensetzung beträgt.

3. Zusammensetzung nach Anspruch 2 oder 3, die Auflösung von mehr als 90 Prozent Rasagilin in 15 Minuten zeigt, wenn sie durch ein Ph.Eur-Auflösungsverfahren unter Verwendung eines Blattrührers bei 50 upm in 500 ml 0,1 N HCl bei 37°C getestet wird.

4. Verfahren zum Herstellen einer pharmazeutischen Zusammensetzung gemäß einem beliebigen der Ansprüche 1 bis 3, wobei das Verfahren Kombinieren von 1 Gewichtsteil Rasagilin-L-Tartrat mit 1-5 Gewichtsteilen L-Weinsäure umfasst.

5. Verfahren nach Anspruch 4, umfassend die Schritte
a) Lösen von 1 Gewichtsteil Rasagilin-L-Tartrat und 1-5 Gewichtsteilen L-Weinsäure in Wasser, und
b) Kombinieren der erhaltenen Lösung mit mindestens einem inerten festen Träger.

6. Verfahren nach Anspruch 4 oder 5, wobei die pharmazeutische Zusammensetzung ein pharmazeutisches Granulat ist.

7. Verfahren nach Anspruch 5 oder 6, wobei die in Schritt a) erhaltene Lösung als eine Granulierungsflüssigkeit während Schritt b) dient.

8. Granulat umfassend 1 Gewichtsteil Rasagilin-L-Tartrat, 1-5 Gewichtsteile L-Weinsäure, und mindestens einen inerten festen Träger.

9. Granulat nach Anspruch 8, erhältlich durch Kombinieren einer wässrigen Lösung von 1 Gewichtsteil Rasagilin-L-Tartrat mit 1-5 Gewichtsteilen L-Weinsäure, mit mindestens einem inerten festen Träger, und Granulieren des Gemisches.

10. Granulat nach Anspruch 8, erhältlich durch Feststoffmischen von 1 Gewichtsteil Rasagilin-L-Tartrat, 1-5 Gewichtsteilen L-Weinsäure, mindestens einem inerten festen Träger, und Nassgranulieren des Gemisches mit Hilfe einer Granulierungsflüssigkeit.

11. Komprimierte Tabletten-Dosierungsform zur oralen Verabreichung umfassend eine Einheitsdosismenge einer Zusammensetzung gemäß einem beliebigen der Ansprüche 1 bis 3.

12. Komprimierte Tabletten-Dosierungsform nach Anspruch 11 umfassend das Granulat gemäß einem beliebigen der Ansprüche 8 bis 10.

## Revendications

1. Une composition pharmaceutique comprenant une partie en poids de L-tartrate de rasagiline, 1-5 parties en poids d'acide L-tartrique, et au moins un excipient inerte pharmaceutiquement acceptable.

2. La composition selon la revendication 1, dans laquelle la quantité en poids relative de tartrate de rasagiline dans la composition pharmaceutique est de 0,2 à 10 pour cent en poids, de préférence 0,5 - 5 pour cent en poids, par rapport à la masse totale de la composition.

3. La composition selon la revendication 2 ou 3, qui présente une dissolution de plus de 90 pour cent de la rasagiline en 15 minutes lorsqu'elle est testée par la méthode de dissolution Ph.Eur utilisant un équipement d'agitation à palettes opérant à 50 tpm dans 500 ml de HCl 0,1 N à 37°C.

4. Une procédé de préparation d'une composition pharmaceutique selon l'une quelconque des revendications 1 à 3, ledit procédé comprenant la combinaison d'une partie en poids de L-tartrate de rasagiline avec 1-5 parties en poids d'acide L-tartrique.

5. Le procédé selon la revendication 4, comprenant les étapes consistant à
a) dissoudre une partie en poids de L-tartrate de rasagiline et 1-5 parties en poids d'acide L-tartrique dans de l'eau, et
b) combiner la solution obtenue avec au moins un support solide inerte.

6. Le procédé selon la revendication 4 ou 5, dans lequel la composition pharmaceutique est un granulé pharmaceutique.

7. Le procédé selon la revendication 5 ou 6, dans lequel la solution obtenue dans l'étape a) sert de liquide de granulation lors de l'étape b).

8. Un granulé comprenant une partie en poids de L-tartrate de rasagiline, 1-5 parties en poids d'acide L-tartrique, et au moins un support inerte solide.

9. Le granulé selon la revendication 8, pouvant être obtenu en combinant une solution aqueuse de 1 partie en poids de L-tartrate de rasagiline avec 1-5 parties en poids d'acide L-tartrique et au moins un support inerte solide, puis en granulant ledit mélange.

10. Le granulé selon la revendication 8 pouvant être obtenu par mélange solide de 1 partie en poids de L-tartrate de rasagiline, 1-5 parties en poids d'acide L-tartrique, et au moins un support inerte solide, puis granulation à l'état humide du mélange en présence d'un liquide de granulation.

11. Une forme galénique de comprimés pour l'administration orale comprenant une quantité de dose unitaire d'une composition selon l'une quelconque des revendications 1-3.

12. La forme galénique de comprimés selon la revendication 11 comprenant le granulé selon l'une quelconque des revendications 8-10.
